# EUROPEAN PATENT SPECIFICATION

(11) **EP 1 231 926 B1**
(45) Date of publication and mention of the grant of the patent: **26.01.2005**
(21) Application number: 00980185.3
(22) Date of filing: 15.11.2000
(51) Int. Cl.: A61K 31/728, A61K 38/27, A61K 38/28, A61K 31/727, A61P 3/10, A61P 5/00, A61P 43/00

(54) **BIOMOLECULAR COMPLEX FORMED BETWEEN HYALURONIC ACID AND A BIOMOLECULE, ITS METHOD OF MANUFACTURE AND MEDICAL USES THEREOF**
BIOMOLEKULARER KOMPLEX GEBILDED AUS HYALURONSÄURE UND EINEM BIOMOLEKÜL, HERSTELLUNGSVERFAHREN DAFÜR UND DEREN THERAPEUTISCHE VERWENDUNG
COMPLEXE BIOMOLECULAIRE FORME ENTRE L'ACIDE HYALURONIQUE ET UNE BIOMOLECULE, SON PROCEDE DE PRODUCTION ET SON UTILISATION MEDICALE

(30) Priority: 15.11.1999 SE 9904121
(43) Date of publication of application: 21.08.2002
(73) Proprietor: Jederström, Gustaf, 113 40 Stockholm (SE)
(72) Inventor: Jederström, Gustaf, 113 40 Stockholm (SE)
(74) Representative: Dahnér, Christer
(86) International application number: PCT/SE2000/002245
(87) International publication number: WO 2001/036656

(56) References cited:
- EP-A2- 0 014 995
- WO-A1-97/37680
- WO-A2-91/04058
- WO-A2-98/01160
- VICTOR A. BLOOMFIELD: 'DNA condensation' CURRENT OPINION IN STRUCTURAL BIOLOGY vol. 1996, no. 6, 1996, pages 334 - 341, XP001009937
- DATABASE WPI Week 9007, Derwent Publications Ltd., London, GB; AN 1990-047916, XP002939890 & JP 2 000 213 A (TAIHOPHARM CO LTD) 05 January 1990
- DATABASE WPI Week 9320, Derwent Publications Ltd., London, GB; AN 1993-164366, XP002939889 & JP 5 097 694 A (DENKI KAGAKU KOGYO KK) 20 April 1993

## Description

The present invention relates to a new biomolecular complex formed by hydrophobic interactions, a method for the preparation of the complex and the use of said complex for the manufacture of pharmacologically active compositions for treating different diseases.

### Background of the invention

Many important drugs comprise or consist of proteins and peptides, such as insulin, hormones etc. The ongoing developments in protein chemistry makes it highly probable that numerous therapeutically effective proteins or peptides are still to be discovered or synthesised. Proteins and peptides are however digested in the gastrointestinal tract, and thus unsuitable for oral administration. Therefor protein and peptide drugs are presently administered parenterally, e.g. by subcutaneous injection.

Various formulations, including liposome encapsulation, have been suggested as delivery vehicles for proteins. In addition to protecting the protein drugs from digestion, their resorption in the body, and consequent transport to the target organs, must be ensured. They must also be delivered to the target organs in an active form. The environment in and the functions of the gastrointestinal tract have hitherto efficiently prevented these goals from being realised. It is for example known that proteins and polypeptides are only absorbed after they have been digested and reduced to short peptides or amino acids.

It can be concluded, that pharmaceutical research is today devoted to improving the ability of drugs to penetrate biological membranes. One approach is to entrap bio-molecules in carrier system such as bioadhesive gels, liposomes or micro-beads. Another approach is to replace peptides and related biological molecules with organic compounds designed for oral delivery and with a molecular mass of less than 500 Dalton. These structures are screened for activation of soluble receptors e.g. in microtitre plates with 96 probes or in an even more dense format. A very large number of structures can easily be evaluated in short time, using techniques like high throughput screening and combinatorial chemistry. However, only a very limited number of potential drug candidates with an active structure has yet been found, although this work started for more than a decade ago.

Hyaluronic acid is a naturally occurring glycosaminoglycan consisting of a linear polymer of repeating units of glucuronic acid and N-acetyl-glucosamine. The molecular weight can vary over a wide range depending on the source. Hyaluronic acid is present in several tissues of animals, and in some specific organs, such as rooster combs, in concentrations high enough for commercial scale extraction. Such tissues contains hyaluronic acid of a wide range of molecular weights and are purified during a complex series of extraction, purification and sterilisation steps. This results in a final product having a high molecular weight within a considerably more narrow molecular weight range. Hyaluronic acid is non-toxic, readily decomposed in the body, and thus suitable for pharmacological use.

One commercial available hyaluronic acid product is HEALON®(Kabi Pharmacia AB, Uppsala, Sweden) which has an average molecular weight of about 4 000 000 Dalton. There are many literature references relating to the use of viscoelastic products of HA in ophthalmologic application and the preparation of such products, including the preparation of chemically modified HA.

### Prior art

In WO 90/05522, hyaluronic acid is mentioned as a slow release carrier in a subcutaneously injected slow release depot, together with a binding protein for e.g. GH or IGF. The active substances are linked to the hyaluronic acid by covalent bonds.

Hyaluronic acid has also been used to improve the adhesion ofbiomolecules to biologic membranes and thus improve the internalisation of biomolecules. However, the main medical use of hyaluronic acid is presently the administration of purified hyaluronic acid (of animal origin or synthesized by use of recombinant cells) directly at sites, where either a mechanical functional barrier is required (viscosurgery, viscoprotection, viscoseparation, e.g. in ophthalmic surgery) or where mechanically dysfunctional tissue can be supplemented (viscosupplementation, viscoaugmentation, e.g. in cosmetic surgery).

Chitosan, a cationic polymer, has been shown to function as a delivery vector for DNA. In WO98/01160, a particulate complex of chitosan and nucleic acid is suggested to function as a novel, non-viral vector for gene-therapy. The chitosan is explained to compact the plasmid DNA into a nanoparticle of between 10 nm and 1 µm and confer upon the compacted material suitable surface characteristics that lead to good adherence of the particle to the surface of the target cell, followed by internalization and expression of the encoded material. This complex is suggested to enchance the expression of nucleic acids in epithelial tissues, such as the GI-tract, the vagina, the rectum, the nasal cavity, the lungs and the buccal cavity. Importantly, the disclosure of WO98/01160 is limited to chitosan, a polyglucosamine extracted from marine evertebrates or the exoscelet of insects. Chitosan is thus a substance foreign to the human body, and it cannot be metabolized by the human body, with exception for the possibility of bacterial degradation possibly taking place in the colon.

In another document, WO97/15330, hyaluronic acid is suggested for use as a DNA carrier for gene therapy to treat abnormal retinal vascularization. In this disclosure, the nucleic acid is not compressed and the hyaluronic acid is used merely as a targeting agent, relying on its ability to bind to cell membranes.

In WO90/09780 it is suggested, that a mixture of insulin and DEAE-dextran or chitosan is delivered to the mucosal surface of a human, e.g. to the mucosal surface of the vagina, the eye, colon or nasal cavity.

### Objectives of the invention

One objective of the present invention is to make available biomolecules capable of efficient uptake in the body of a mammal, and in particular after oral administration, and exhibiting therapeutic effect after oral administration.

Another objective of the present invention is to make available a biomolecule capable of passing the brain-blood-barrier.

Another objective is to make available a hormone formulation for oral administration, said formulation exhibiting therapeutic effect *in vivo* after oral administration.

Still another objective is to make available an insulin formulation for oral administration, said formulation exhibiting therapeutic effect *in vivo* after oral administration.

Still another objective is to make available a growth hormone formulation for oral administration, said formulation exhibiting therapeutic effect *in vivo* after oral administration.

Still another objective is to make available a heparin formulation for oral administration, said formulation exhibiting therapeutic effect *in vivo* after oral administration.

A further objective of the present invention is to make available a medicament for delivering therapeutically active biomolecules orally, obliterating the need for parenteral administration, e.g. injections.

An objective of the invention is to make available novel biomolecular complexes for use in the manufacture of a medicament for treating different diseases, wherein a therapeutically active biomolecule is delivered orally.

### Summary of the invention

The present invention solves the above problems by making available a new complex between a pharmacologically active biomolecule and hyaluronic acid involving hydrophobic interaction. The present invention also makes available novel therapeutic formulations and their use in the manufacture of a medicament for therapeutic application.

A preferred embodiment of the present invention is a complex between a biomolecule and hyaluronic acid, characterized in that the complex is formed by means of hydrophobic interactions between the components.

In another preferred embodiment of the present invention, said complex between a biomolecule and hyaluronic acid is characterized in that the biomolecule is rendered substantially hydrophobic and enclosed in hyaluronic acid.

In another preferred embodiment of the present invention, said complex is characterized in that the biomolecule's size is reduced, preferably to less than about 10 % of its original size.

In yet another preferred embodiment of the present invention, said complex is characterized in that said biomolecule is a peptide.

In yet another preferred embodiment of the present invention, said complex is characterized in that said biomolecule is a protein.

In yet another preferred embodiment of the present invention, said complex is characterized in that said biomolecule is a glycoprotein.

In yet another preferred embodiment of the present invention, said complex is characterized in that said biomolecule is a polynucleotide.

In yet another preferred embodiment of the present invention, said complex is characterized in that said peptide is chosen among the following groups of substances: hormones, neuropeptides, signal peptides, peptide antibiotics, peptide antigens, antibodies and naturally occurring or synthetic amino acid polymers.

In yet another preferred embodiment of the present invention, said complex is characterized in that said peptide is chosen among the following substances: insulin, growth hormone, human growth hormone, glucagon, corticotropin, oxytocin, bradykinin, thyrotropin-release factor and thyrotropin, enkephalins, and peptide antibiotics.

In yet another preferred embodiment of the present invention, said complex is characterized in that said polynucleotide is chosen among the following: plasmides, DNA, RNA, cDNA, mRNA, recombinant DNA, recombinant RNA, and combinations thereof.

In yet another preferred embodiment of the present invention, said complex is characterized in that said hyaluronic acid is hyaluronic acid having a molecular weight of less than about 400 kDa, preferably in the interval of 60 to 360 kDa.

In yet another preferred embodiment of the present invention, said complex is characterized in that said hyaluronic acid is hyaluronic acid having a molecular weight of about 150 kDa.

Another embodiment of the present invention is a medicament for oral delivery of biomolecules characterized in that the biomolecule is incorporated in the hyaluronic acid complex defined above, and the resulting complex administered orally to a patient.

Another preferred embodiment of the present invention is a method for manufacturing a complex of a biomolecule and hyaluronic acid, characterized in that the method comprises the following steps:
a) dissolving hyaluronic acid in an electrolyte solution,
b) adding the biomolecule to the dissolved hyaluronic acid, and
c) dialysing the solution at about neutral pH.

In another preferred embodiment of the present invention, said method is characterized in that the acid in step a) is chosen among hydrochloric acid, sulphuric acid, phosphoric acid and acetic acid and the pH is in the range of 1.0 to 3.0.

In yet another preferred embodiment of the present invention, said method is characterized in that the electrolyte solution of step a) contains cations chosen among NH₄⁺, K⁺, Na⁺, Ca²⁺, Mg²⁺, Zn²⁺, Fe²⁺, Fe³⁺ and anions such as sulphate, nitrate, chloride, hydroxide and acetate.

In yet another preferred embodiment of the present invention, said method is characterized in that the pH in step c) is in the interval of 5.5 to 7.5, preferably in the interval of 6.0 to 7.0.

In yet another preferred embodiment of the present invention, said method is characterized in that said hyaluronic acid is hyaluronic acid having a molecular weight of less than about 400 kDa, preferably in the interval of 60 to 360 kDa.

In yet another preferred embodiment of the present invention, said method is characterized in that said hyaluronic acid is hyaluronic acid having a molecular weight of about 150 kDa.

Another preferred embodiment of the present invention is the use of insulin for the manufacture of a complex with hyaluronic acid, for use as a medicament for the treatment of diabetes.

Another preferred embodiment of the present invention is the use of growth hormone for the manufacture of a complex with hyaluronic acid, for use as a medicament for the treatment of growth hormone deficiencies.

Another preferred embodiment of the present invention is the use of growth hormone for the manufacture of a complex with hyaluronic acid, for use as a medicament for the prevention of transplant rejections.

Another preferred embodiment of the present invention is the use of heparin or bioactive derivatives thereof for the manufacture of a complex with hyaluronic acid, for use as a medicament.

According to the invention, peptides are reversibly compressed, e.g. from a size of 2.4 nm to less than 0.3 nm, thus improving their ability to penetrate across biological membranes e.g. the gut wall. A plasmid-DNA construct has been reversibly compressed from 87 nm to less than 10 nm, improving the DNA-structure for free passage through the nuclear pore. Similarly, inventive complexes with growth hormone, insulin and heparin have been produced and tested. One way of producing these new hydrophobic complexes is by pH-changes, by elimination of molecular charge and bonds, by evacuation of water and ions, and finally stabilisation with hyaluronan.

Full biological effect of the compressed peptides was demonstrated in biological assays and in *vivo.* The size and the durable hydrophobic properties of the peptide, obtained at pH 6, suggest an improved oral bioavailability. The compression of a plasmid-DNA complex from about 87 nm to about 7.5 nm suggests the possibility of obtaining an improved transfection efficiency.

These reversibly compressed complexes can be administrated by new routes and thereby avoid the need of parental administration. The new routes made available for these complexes include oral, pulmonary, nasal, and topical administration, and intra cellular trafficking.

Avoiding parenteral administration will make drug application more convenient, e.g. for out patients, self-medicating patients, and especially for children and the elderly. A drug according to the invention will also be easier to handle, which will result in an improved compliance.

### Short description of the drawings

The invention will be disclosed in closer detail in the following description, examples and attached drawings, in which
Fig. 1 shows the hydrodynamic radius in nm as a function of hyaluronic acid concentration in ug/ml in different dilutions;
Fig. 2 shows the kinetic blood glucose profile in six rats, receiving an insulin-Hy complex (2.44 mg/ml, 9.5 U) by subcutaneous injection;
Fig. 3 shows the kinetic blood glucose profile in six rats, receiving an insulin reference (4.56 mg/ml, 17.8 U) by subcutaneous injection;
Fig. 4 is an AFM (Atomic Force Microscopy) photography of a sample of the insulin-Hy complex at a concentration of 134 U insulin / ml, showing clearly visible dots;
Fig. 5 is an AFM photography of a sample of the insulin-Hy complex at a concentration of 40.4 U insulin / ml, showing clearly visible dots;
Fig. 6 shows the blood glucose levels in three rats, after oral administration of an insulin-Hy complex (1 ml. 28 U/ml);
Fig. 7 shows the blood glucose levels in three rats, after oral administration of an insulin-Hy complex, where rat no. 1 and no. 2 were on starvation diet, and rat no. 1 received 0.2 ml 134 U/ml or 27 U, rat no. 2, 1 ml 62 U and rat no. 3 was on normal diet and received 1 ml, 62 U insulin;
Fig. 8 shows the blood glucose levels in two rats, after subcutaneous administration of the insulin reference (0.065 ml, 4 mg/ml, 6.8 U); and
Fig. 9 shows the weight increase in % in Hx rats receiving the rhGH complex, compared to non-treated rhGH and placebo.

### Description

The present invention concerns a method for forming a complex between hyaluronic acid and a biomolecule involving hydrophobic interactions, the oral administration of these biomolecules, and various methods involving the administration of these biomolecules. In case the necessary hydrophobic sites are not available for complex formation, the biomolecule is rendered at least partially hydrophobic by a process herein called compression. The invention thus makes available a method for the manufacture of compressed biomolecules and complexes according to the invention.

In the description of the invention, the following terms will be used:

The term "biomolecule" is used in the description, examples and claims to define all molecules, synthetic and natural, exhibiting an effect *in vivo.* Examples of biomolecules include peptides, proteins, glycoproteins, glucosaminoglucans, and sugars. The term "pharmacologically active substance" is used as a synonym to the above.

The term "peptide" includes hormones, neuropeptides, signal peptides, peptide antibiotics, peptide antigens and other naturally occurring or synthetic amino acid polymers.

Among naturally occurring, recombinant and synthetic peptides suitable for use according to the present invention are hormones, such as insulin, growth hormone, human growth hormone, glucagon, corticotropin, oxytocin, bradykinin. thyrotropin-release factor and thyrotropin, enkephalins, and peptide antibiotics.

The term "proteins" includes enzymes, transport proteins. nutrient and storage proteins, contractile or motile proteins, structural proteins, defence proteins, regulatory proteins and other proteins, according to a text book definition.

Among naturally occurring, recombinant and synthetic proteins suitable for use according to the present invention are enzymes, such as oxidoreductases, transferases, hydroiases, lyases, isomerases and ligases; transport proteins such as metalloproteins, hemoproteins, phosphoproteins, glycoproteins and lipoproteins; defense proteins, such as proteins taking part in the immune defense, e.g. immunoglobulins, antibodies, proteins taking part in tissue repair, e.g. fibrinogen, thrombin etc; regulatory proteins, such as hormones, growth factors, e.g. epidermal growth factor (EGF), nerve growth factor (NGF), fibroblast growth factor (FGF), platelet derived growth factor (PDGF), erythropoietin, cytokines, lymphokines, including interleukins (IL1, IL-2 etc.) and interferons.

The term "hydrophobic binding" is used in this context to define bindings that are essentially of hydrophobic nature and influenced by long range forces, such as van der Waals forces between hydrophobic parts of a biomolecule and the hydrophobic parts of a molecule surrounding the biomolecule.

The terms "compression" and "compressed" as in "compressed biomolecule" are used in this text to define a reversible size reduction, performed on the biomolecules according to the inventive method.

The term "reversible" in this context means that the biomolecule retains or regains its biologic effect in vivo after compression and administration to a mammal, in particular after oral administration to a mammal.

By "hyaluronic acid" is meant hyaluronic acid both in protonated and non-protonated form, irrespective of origin. The invention thus encompasses the use of hyaluronic acid of both animal and bacterial origin, for example hyaluronic acid produced by genetically modified organisms. In general, the invention encompasses the use of all hyaluronic acid derivatives, which do not compromise the physiological properties associated with hyaluronic acid. Further, the invention encompasses the use of derivatives and complexes which can be converted to hyaluronic acid in the human gastrointestinal tract. The hylauronic acid molecules exist normally in the form of loops. but the present inventor has surprisingly found that it can be straightened in the presence of protons, H+. Thus, when HCl is added to a solution of hyaluronic acid of a predetermined molecular weight, the molecules will become straight, and positively charged.

Preferably hyaluronan (Hy) with a molecular weight in the range of 80-360 kDa is used, preferably Hy having a molecular weight of about 150 kDa.

An optimal compression (=size) and hydrophobic properties of plasmid (preferabbly less than about 10 nm) and peptides (preferably less than about 0.3 nm) are found by elimination of charge and molecular bonds by addition of HCl to pH < 2 and of different ions. These ions are cationic ions: NH₄⁺, K⁺, Na⁺ and anionic ions: sulfates, chloride. carbonates, acetates.

A stable complex/polymer is formed by Hy surrounding the plasmid, the peptides respectively when Hy changes its structure back to a curled structure when pH is changed to pH 6. It is then possible to dilute the solutions to the desired strength.

The resulting polymer complex can be produced in the form of a precipitate. a colloidal solution or a true solution, preferably in the form of a colloidal solution. The complex can also be prepared in the form of a lyophilised powder.

Another embodiment of the present invention is a method for the production of the hydrophobic biomolecular complex. The method includes the steps of:
a) solubilisation of the polymer and solubilisation of the polar biostructure by addition of an acid;
b) collapsing the polar biostructure by addition of an electrolyte by passing the polar biostructure over polar biostructure's point of collapse and changing the hydrodynamic radii to a minimum compressed size; and
c) dialysing the biomolecular structure, thereby obtaining a compressed hydrophobic complex with the polar groups buried and the polymer surrounding the biostructure.

In a preferred embodiment of the inventive method the acid in step a) is hydrochloric acid, sulphuric acid, phosphoric acid or acetic acid, and the pH is less than 3, preferable in the range of 1.0 to 2.5.

In another preferred embodiment of the inventive method the electrolyte of step b) contains cations such as NH₄⁺, K⁺, Na⁺, Ca²⁺, Mg²⁺, Zn²⁺, Fe²⁺, Fe³⁺ and anions such as sulfates. chloride, and acetates.

In another embodiment of the present invention the hydrophobic biomolecular complex is used as a medicament.

It is surprising that hyaluronic acid can be used in the manner described in the present description and examples, as one problem encountered with hyaluronic acid is its extreme and undesired adsorption of peptides. This is a well documented problem in the production of hyaluronic acid for medical purposes, where peptide contamination or oligonucleotide contamination (e.g. DNA, RNA) renders the product impossible to use, as the remaining peptides are pyrogenic or irritating for the patient.

A problem specific for the administration of insulin is the very narrow dose interval tolerated by the patients. It is therefor highly surprising that the complex of hyaluronic acid and insulin according to the present invention both gives effect at low doses and is well tolerated also at very high doses, as shown in the *in vivo* experiments.

The inventive complexes of biomolecules and hyaluronic acid are also suitable for parenteral administration. In this application they have the advantages of sustained effect and higher dose tolerance. Further, the need of chemically modifying the substances - other than forming the complex with hyaluronic acid - is avoided.

A complex according to the invention is administered to the patient orally, in the form of a suspension, a capsule, a tablet, an enteric coated capsule or another oral delivery form, including vechicles and adjuvants commonly used for oral preparations.

A complex according to the invention is administered to the patient transdermally, in the form of a suspension, a gel or paste, or another oral delivery form, including vechicles and adjuvants commonly used for transdermal preparations.

A complex according to the invention is administered to the patient nasally, in the form of an aerosole of liquid or solid particles, or another nasal delivery form, including vechicles and adjuvants commonly used for nasal preparations. Similarly to nasal administration, the inventive complex can be administered orally, by inhalation, in the form of an aerosole of liquid or solid particles, or another delivery form, including vechicles and adjuvants commonly used for preparations intended for inhalation.

Although the inventive complex is specially suited for oral delivery, it may also be subcutaneously or intramuscularely administered. When administered parenterally, the inventive complex will exhibit better bioavailability, and it may function as a sustained release preparation.

The use of hyaluronic acid has a special advantage in that endogenous pathways are readily available to metabolize the hyalyuronic acid. Further, as the human body has a high capacity to metabolize large quantities of hyaluronic acid, the small quantities applied therapeutically according to the present invention do not add significantly to the systemic metabolic burden.

The inventive complex and methods also have an considerable advantage in that presently available pharmaceuticals, approved for human use, can be used more efficiently and - in the case of oral administration - delivered via a novel route of administration, hitherto unavailable for these pharmaceuticals. As both hyaluronic acid and the pharmaceuticals suggested for use according to the present invention are thoroughly investigated, the tests required for the new complexes can be minimised.

An advantage of the present invention is further, that the cost of production for most of these novel drugs will be less than the cost for parenteral drugs, since there are less strict requirements for sterile production of drugs administered orally.

### Examples

### Example 1: The influence of ph-change on hydrodynamic radius

A substantial buffer capacity in aqueous Hy-solution was observed when diluted acids were added. Strong acid-solutions should be avoided due to breakdown of the polymer Hystructure and also to loss of the acetyl-group. The time for studies of Hy with different plasmid/peptides should therefore be limited to around 30 min.

The particle size of Hy was studied upon pH-changes. Dynamic light scattering, 500 mV, was determined for three concentrations of Hy 90, 30 and 10 µg/ml. Malvern, ZetaMaster S, version PCS: v 1.26 was also used to determine pH and ξ-potential

The resulting relaxation time distributions are essentially single-modal under all conditions. The peak width decreases systematically with increasing concentration due to a greater signal to noise ratio.

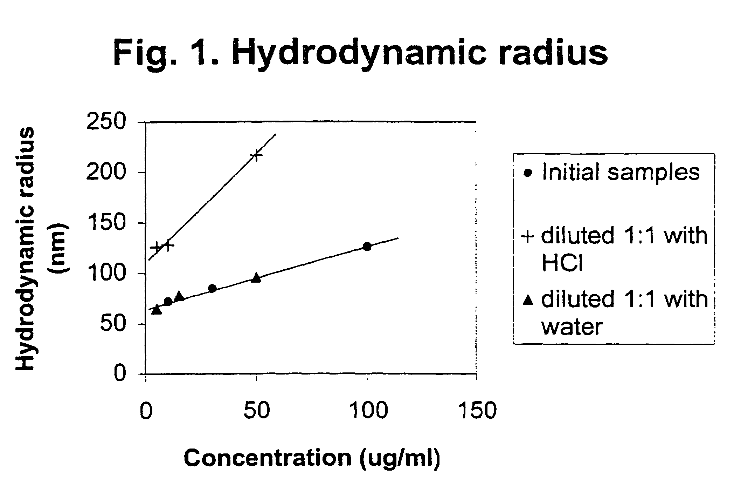

Figure 1 shows the hydrodynamic radius (Rn) in nm (0 - 300) as a function of concentration of Hy in µg/ml. The calculated hydrodynamic radii fall on the same line for the undiluted samples and those diluted with water. Those samples diluted with acid have considerably larger particle size. The true particle sizes in contrast to the apparent values which are influenced by interactions at finite concentrations are 65 nm (water solutions) and 105 (acid diluted). It is noteworthy that the scattered intensities are the same for the undiluted and water diluted samples, but about the double in value for the acid-diluted. For example, 11 kHz for the water-diluted and 26 kHz for the acid-diluted solutions. This is consistent with an increase in particle dimensions in acid-diluted solutions of Hy. This is indicative for that the Hy structure upon strong acid addition is stretching out from a curling cylinder to straight line.

Hy with a molecular weight of 150 k Dalton becomes positively charged (3.1-16.9 mV) when pH changes from pH 6.5 to 1.5. Particle size measurements by dynamic light scattering 500 mV indicate that the Hy structure is stretched out from a curled cylinder to a straight line upon addition of a strong acid. pH is changed from 6.5 to 1.5 and the hydrodynamic radii is almost doubled or changed from 65 to 105 nm.

### Example 2. Preparation of a complex of insulin and hyaluronan for oral and parenteral administration

The aim of this study was to evaluate if the insulin complex had biological effect, the duration of such an effect and the dose required to obtain a significant decrease in blood glucose level.

The substances used were:
- Human recombinant insulin (Roche) Lyophilizate, sterile. specific activity >26 U /mg.
- Hyaluronan (Hy), molecular weight 150.000 Dalton, prepared by fractionated hydrolysis. The mean molecular weight was determined by Size Exclusion Chromatography (SEC).

The following preparations were used for parenteral administration in streptozotocin diabetes rats:
1) A placebo solution, consisting of an aqueous isotonic sodium chloride solution (0.9%)
2) A complex of insulin-Hy (2.44 mg / ml), dose 3 x 0.05 ml or 9.5 U / rat and day
3) Human insulin, (rDNA), dose 3 x 0.005 ml or 17.8 U per rat and day
4) Human insulin (rDNA) Ultartard, 100 IE / ml (3.5 mg / ml)), from Novo Nordisk, dose 100 µl suspension or 10 IE per rat and day

The preparations were made under sterile conditions.

### Help solutions were:

Aqua Sterilisata (Kabi Pharmacia AB Sweden); human albumin solution, 200 mg/ml (Pharmacia & Upjohn); isotonic sodium chloride solution (0.9% l, pH 6.0, Kabi Pharmacia AB Sweden); 1 M sodium sulfate: 1 M and 6 M hydrochloride acid; 5 M sodium hydroxide; isotonic Tris buffer (10 mM pH 6.5, sodium chloride 0.9%), and hypotonic Tris buffer (10 mM pH 6.5).

Before use, all solutions were filtered through a 0.22-micron filter.

### 2.1 Preparation of an insulin complex according to the invention

Different molar ratios of Insulin: Hy were used in the following production examples: a molar ratio of 12:1 for "Preparation I " and 15:1 for "Preparation II".

### Preparation I

A defined amount of insulin (45 mg) was dispensed into a sterilized glass bottle. Hy (92 mg) was slowly dissolved in sodium sulfate (6.5 ml, 1 M) in a sterile glass container. The mixing was interrupted when the solution became transparent. 1 M hydrochloride acid was then added to the mixture of Hy to pH 1.51 - 1.58. The insulin (45 mg) was then added to the Hy-solution and dissolved. The mixture (pH 1.5) was gently dispersed and transferred into dialysis bags.

### Preparation II

A defined amount of insulin (45 mg) was dispensed into a sterilized glass bottle. Hy (78.9 mg) was slowly dissolved in sodium sulfate (7.0 ml, 1 M) in a sterile glass container. The mixture was allowed to become transparent. 1 M hydrochloride acid was added to reach pH 1.6. Insulin (45.1 mg) was then added to the Hy solution together with hydrochloric acid (0.2 ml, 1 M)and sodium sulfate (0.8 ml, 1 M) to properly dissolve the insulin (pH 1.80). The mixture was transferred into dialysis bags.

### 2.2 Preparation of an insulin reference solution

### Preparation III

Insulin (40mg) was dissolved in an isotonic sodium chloride solution (8.0 ml) in a sterilized glass bottle. The pH was adjusted to 1.8. The resulting transparent solution was dispensed into dialysis bags.

### 2.3 Dialysis procedure for an insulin-Hy complex according to the invention

For the dialysis procedure 6 dialysis bags (Spectra Pore R MWCO 6-8000) each with a volume of 4 ml were prepared. The three preparations (3x2) were placed in a beaker containing a buffer solution. The dialysis procedure was performed according to Table 1 below.

Buffer solutions: an isotonic Tris buffer (10 mM, pH 6.5, 0.9% NaCl) in the following designated "isotonic", and a hypotonic Tris buffer (10 mM, pH 6.5), in the following designated "hypotonic" were respectively used to dialyse the preparations.

The dialysis medium was changed after different time intervals. The pH of the medium outside the dialysis bags was measured after each dialysis period and the preparations were inspected. The pH did not exceed pH 6.5 at any point. After the dialysis, the content of the dialysis bags were pooled for each preparation and transferred into sterile glass bottles which were closed and placed at + 4°C. These solutions were refereed to as "stock solutions" of the final insulin-Hy complexes according to the invention, and the "insulin reference".

**Table 1.**

| **Dialysis schedule for insulin-Hy complex** | | |
|---|---|---|
| Change of solution in the dialysis procedure | | |
| Accumulated dialysis time (h) | Ratio of added medium for dialysis | pH outside the dialysis tubes |
| 0 | 50% isotonic | 6.5 |
| | 50% hypotonic | |
| 15 | 100% hypotonic | 3.79 |
| 16.5 | 50% isotonic | 3.8 |
| | 50% hypotonic | |
| 17 | 50% isotonic | 6.0 |
| | 50% hypotonic | |
| 18.5 | 50% isotonic | 5.8 |
| | 50% hypotonic | |
| 19.5 | 100% isotonic | 5.7 |
| 46.5 | 100% isotonic | 6.48 |
| 69.5 | 100% isotonic | 6,5 |
| 88 | 100% isotonic | 6.5 |
| | | |
| End | End | End |

The first stock solution "Preparation I" had a volume 9.5 ml and pH 6.4, and appeared as a transparent solution/gel with some cloudiness/precipitate. Amino acid analysis was performed, and indicated a concentration of 5.16 mg/ml insulin or 134 U /ml. Preparation I was used for
i) Atomic Force Microscopy (AFM), and
ii) oral administration to rats (dose 0,2 ml, 27 U).

The second stock solution "Preparation II" had a volume of 15.5 ml and pH 6.4, and appeared as a transparent solution with some cloudiness/precipitate. Amino acid analysis was performed, and indicated a concentration of 2.44 mg/ml insulin or 63 U /ml. Preparation II was used for
i) subcutaneous injections in rats. and
ii) kinetic studies in six rats. subcutaneous injection.

The third stock solution "Preparation III" had a volume of 8.2 ml. and pH 6.4, and appeared as a transparent solution. Amino acid analysis was performed, and indicated a concentration of 4.57mg/ml insulin or 118.9 U/ml. Preparation III was used as the insulin reference solution.

### Example 3: Subcutaneous administration of an insulin- Hy complex, Preparation II

The composition of the insulin-Hy complex was:

| | |
|---|---|
| Preparation II | 6.080 ml |
| Human albumin | 0.27% |
| Concentration: | 2.44mg/ml insulin or 63 U /ml |

The composition of the insulin reference was:

| | |
|---|---|
| Preparation III | 4.0 ml |
| Human albumin | 0.27% |
| Concentration: | 4.57mg/ml insulin or 118.9 U /ml |

Ultratard 100 IE / ml_Novo Nordisk Dose 100 µl or 10 U.

As placebo, a physiological sodium chloride solution (0.9% NaCl, pH 6.0, Kabi Pharmacia AB, Sweden) was used.

Four groups of six rats received subcutaneous injections with the insulin-Hy complex (0.05 ml x 3), the insulin reference solution (0.05 ml x 3), Ultratard 100 IE / ml dose 100 µl or 10 IE, and placebo respectively.

The blood glucose levels were followed for 9 hours. The blood glucose values were for Ultratard 7.58 mMol/l, for the insulin reference solution 8.52 mMol/l, for the insulin-Hy complex 9.78 mMol/ml, and for placebo 20.36 mMol/ml.

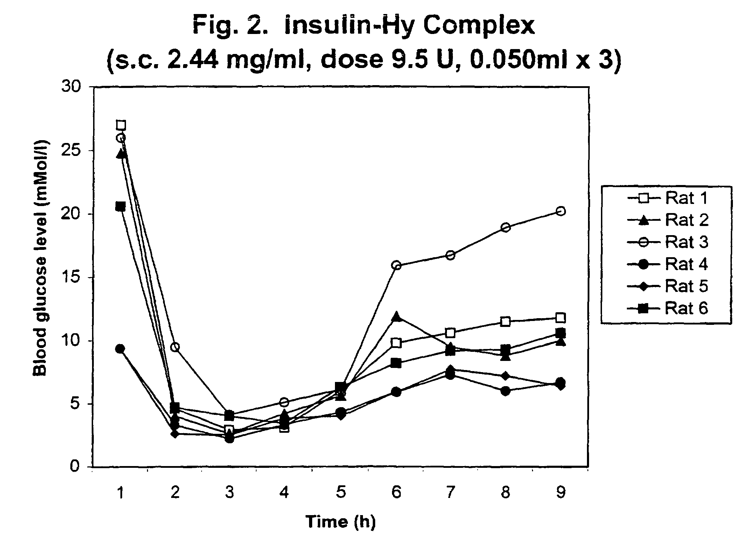

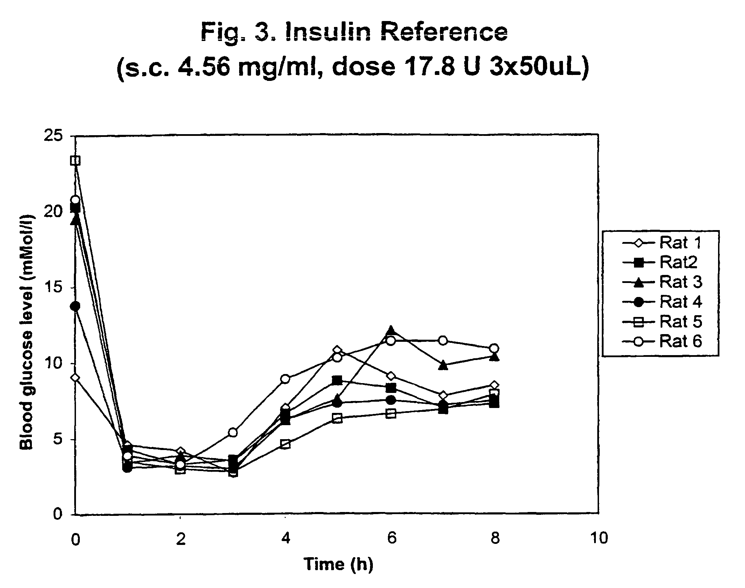

The kinetic behaviour was studied for the insulin-Hy complex 2.44 mg /ml, given 0.05 ml x 3, and for the insulin reference solution 4.56 mg/ml given 0.05 ml x 3. The blood glucose levels were followed after subcutaneous injections. See Figure 2 and 3.

These studies suggest full biological activity of Insulin-Hy Complex according to the invention.

### Example 4: Characterization of the polymer Hy-Insulin complex by AFM (Atomic Force Microscopy)

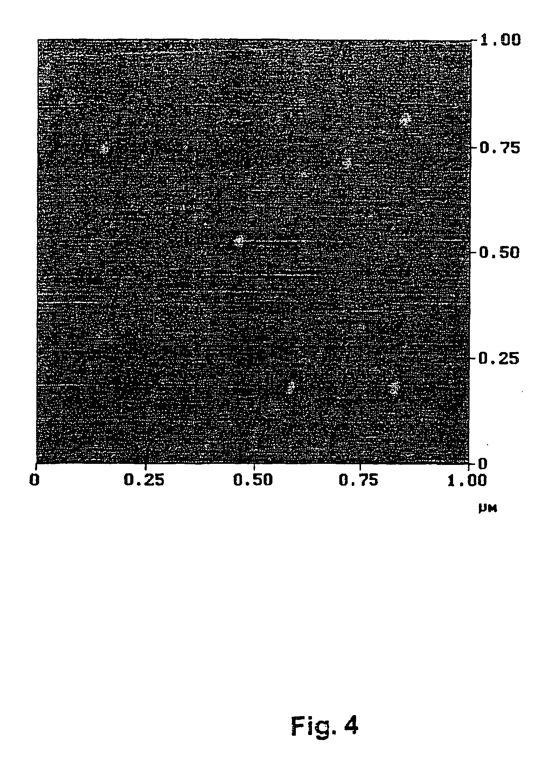

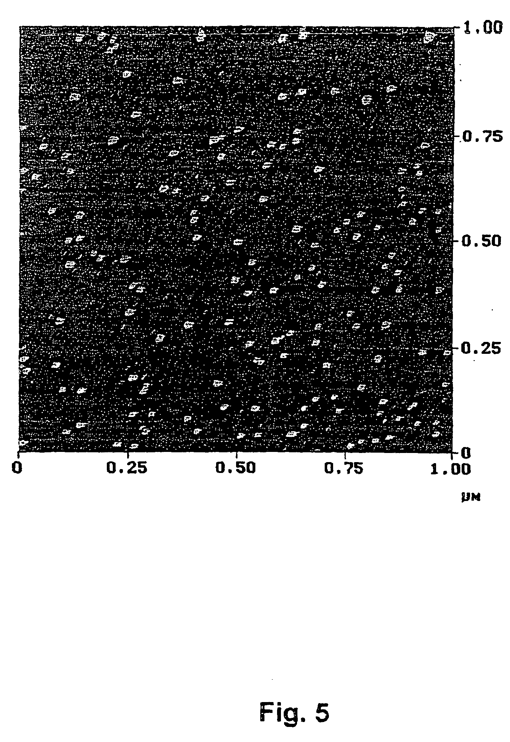

Diluted solutions of the insulin reference, hyaluronan (Hy) 150 000 Dalton and the insulin-Hy complex, were placed on mica chips and examined by tapping AFM. It was found under the conditions used, that neither the insulin reference nor Hy itself resulted in any significant images. Samples of the insulin-Hy complex prepared as described above however gave clearly visible dots at a concentration of insulin 134 U/ml (See Fig.4) and at a concentration of insulin 40.4 U/ml (See Fig. 5). This indicates that the complexes exist, can be isolated and identified, and that the complex is stable under the conditions used of storage.

### Example 5: Oral administration of the insulin-Hy complex

The objectives of these studies were to determine the dose level and concentration of the insulin-Hy complex in streptozotocin diabetic rats by finding a measurable absorption of orally administered insulin in form of the inventive insulin-Hy complex. The blood glucose level was observed in order to detect a possible effect of the complex. The Sprague Dawley rats were fed 1ml of the samples by a tube in the stomach.

### 5.1 Preparation of insulin for oral administration

For oral administration in streptozotocin diabetic rats (streptozotocin i.v.; 40-mg/kg bodyweight rats) the following preparations were made:
Insulin-Hy complex: 0.4, 3.2 and 28 U / ml. Oral dose given 1 ml - 0.4, 3.2, 28 U respectively.
Reference insulin solution for subcutaneous injection, recombinant human insulin 3.86 mg/ml or 33.4 U / ml. Subcutaneous dose given 65 µl - 6.5 U

The insulin-Hy complexes were prepared essentially as described above (Example 2) and aseptically transferred into injection vials. Human albumin 0.27% was omitted in the oral preparations.

The solutions of 0.4 and 3.2 U/ml insulin-Hy complexes were transparent. The 28 U/ml solution was turbid. The content of the solutions was assayed using amino acid analysis.

The mixtures were contained in the vials at 8 °C prior to oral administration. The stability of the solutions at 8 °C was found to be at least 2 months (no physical or biological changes of the preparations were observed).

The insulin-Hy complex 0.4, 3.2 and 28 U/ml were given orally to streptozotocin diabetic rats (streptozotocin i. v.; 40 mg/kg bodyweight).

### 5.2 Reference preparation

Recombinant human insulin 3.93 mg / ml or 102 U / ml (Dose given 65 µl = 6.6 U) 3.0 ml was aseptically prepared. The pH was adjusted to 5-6 and the solution was transparent.

Composition:

| | |
|---|---|
| Recombinant human insulin | 11.8 mg |
| Sterile water | 1600 µl |
| Human albumin | 50 µl |
| Physiological sodium chloride | 1360 µl |

### 5. 3 Dose level oral administration / Rats on normal diet

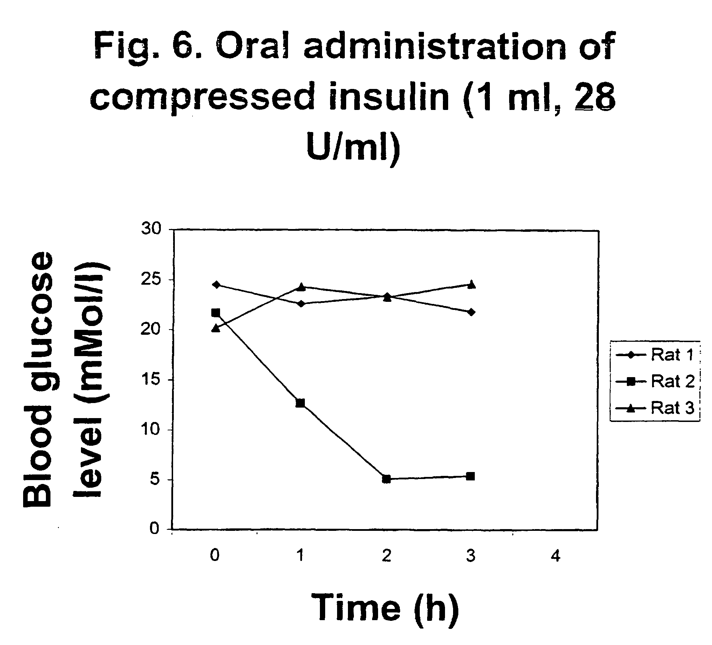

The blood glucose level in rats given the insulin reference orally did not change significantly. At a dose level of 28 U/l, one of three rats responded with a decrease in blood glucose level (See Fig. 6). The dose level for oral administrated Insulin Complex was estimated to be 28 U. This dose gave a decrease in glucose level for more than three hours. (The measurements were stopped after 3 hours).

### 5.4 Dose level subcutaneous administrations

Insulin reference (4.56mg/ml, 0.05m) x 3): 17.8 U resulted in a decrease in blood glucose level from 20.3 mMol/l to 8.5 mMol/l for more than eight hours.

Ultartard 100 IE/ml (3.5 mg / ml) Insulin human (rDNA), Novo Nordisk, dose 100 µl suspension or 10 U): 10 U resulted in a decrease in blood glucose level from 20.3 mMol/l to 7.6 mMol/l for more than eight hours.

Insulin-Hv complex (2.44 mg / ml 0.05x3): 9.5 U resulted in a decrease in blood glucose level from 20.3 mMol/l to 9.7 mMol/l for more than eight hours.

### 5.5 Rats on starvation diet

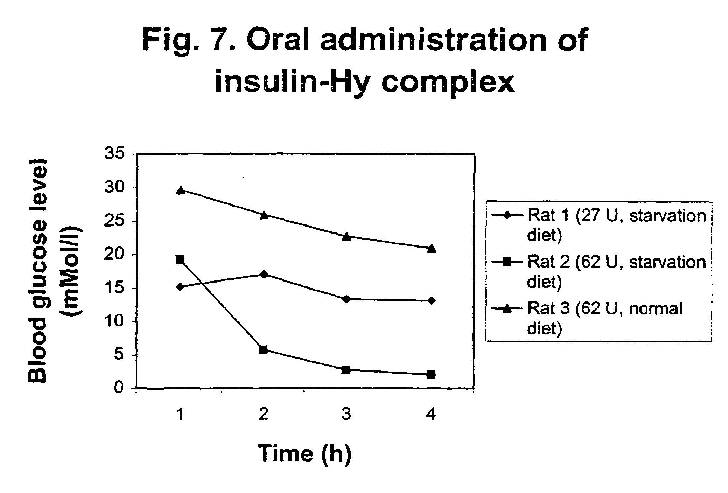

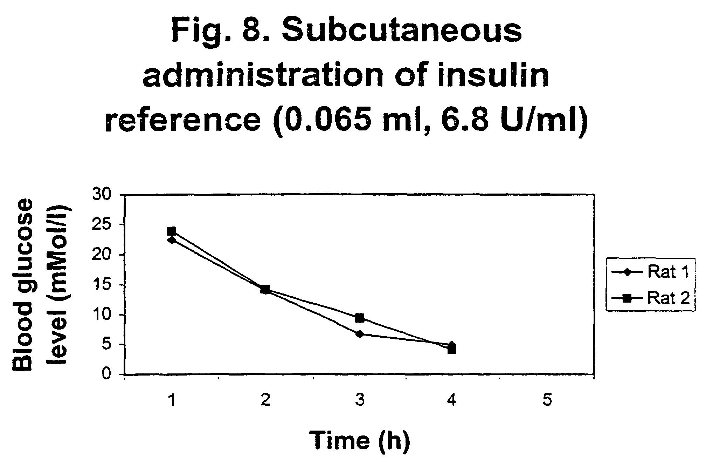

Rats were starved during one night, but supplied with water. On the morning the blood glucose level was determined. Three rats were studied for oral absorption. Two of them were on starvation diet and orally treated with 26.8 U, 62 U respectively. One of them, not on diet, was orally treated with 62 U. Two reference rats were given subcutaneous injections. All rats responded with a decrease of the blood glucose level, as seen in Figures 7, and 8.

The differences between the results may be explained by the activity pattern of the rats, considering the fact that the insulin-Hy complex was orally administered in the morning to rats with a stomach filled with food.

Six streptozotocin diabetic rats were orally administered with Insulin-Hy Complex at a dose level of 28 U or more. Four of them responded with a decrease in blood glucose level. Two of them did not react. This suggests that a satisfactory degree of oral absorption of insulin was obtained when given in the complexed form according to the invention. The variations in the results also suggests, that various parameters influencing the tests, such as the diabetic rat's activity period, the food content of their gastrointestinal tract etc should be standardized. The studies and results obtained in rats are however highly relevant and can be transferred to humans suffering for type 1, and type 2 diabetes.

### Example 6: Preparation of beparin-Hy complex according to the invention

Heparin (16.1 mg) having a molecular weight of 7.500 Dalton (Sigma) was dispensed in a sterilized glass bottle. Hyaluronan (30.0 mg) was slowly dissolved in sodium sulphate (1,2 ml, 1 M) in a sterilized glass container, and stirred until transparent. Hydrochloride acid (0.85 ml, 1 M) was added to pH 1.48. The heparin was then added to the hyaluronan solution and dissolved. The mixture was transferred into dialysis bags.

### 6.1 Dialysis procedure for the heparin-Hy complex according to the invention

Two dialysis bags (Spectra Pore R MWCO 6-8000) each with a volume of 4 ml were prepared. The preparations (1x2) were placed in a beaker containing a buffer solution. The dialysis procedure was performed according to the schedule given below, in Table 2.

Buffer solution: A hypotonic Tris buffer (10 mM, pH 7.5) designated "hypotonic", was used to dialyse the preparations.

**Table 2.**

| **Dialysis schedule for heparin-Hy complex** | | |
|---|---|---|
| Change of solution in the dialysis procedure | | |
| Accumulated Dialysis Time (h) | Ratio of added medium for dialysis | pH outside the dialysis tubes |
| 7 | 100% hypotonic | 3.79 |
| 61 | 100% hypotonic | 6.7 |
| 85 | 100%hypotonic | 6.7 |
| 88 | 100% isotonic | 6.7 |
| End | End | End |

The dialysis medium was changed after different time intervals. The pH of the medium outside the dilysis bags was measured after each dialysis period and the preparations were inspected. The pH did not exceed pH 6.5 at any point. After the dialysis, the content of the dialysis bags were pooled, and transferred into a sterile glass bottle which were closed and stored at 4°C.

The dialysed heparin-Hy complex had a volume of 1.05 ml, and pH 7.2, and appeared as a transparent solution. The preparation was used for particle sizing in a Malvern ZetaSO apparatus, version PCS v1 .29.1. The size by volume was determined to be 1272 nm for this preparation.

The above example shows the feasibility of the inventive method also for heparin.

### Example 7: Plasmid-Hy complex

This example illustrates the mechanisms of Hy in obtaining a durable compact plasmid-DNA-structure. The gene expression of the plasmid is encoded for Chloramphenicol Acetyl Transferace. CAT.
Solution 1: Plasmid pRc/CMV-CAT, double strained closed-ring structure ∼6400 base pair, molecular weight 4 250 000 Dalton 200µL was used in the concentration of 130 µg/ml.
Solution 2: 10.4 mg Hy molecular weight 150 000 Dalton was dissolved in 100 ml water in a concentration of 104 µg/ml.
Solution 3: 2 M NaCl
Solution 3.1: 0.15 M NaCl was used
Solution 4: 1 M NaOH
Solution 5: 1 M HCl
The aimed final solution: 5 00 µl was formulated to contain

| | | | Conc. | | |
|---|---|---|---|---|---|
| | | | 500 µl in µg/ml | | (µg/µl) |
| pRc/CMV-CAT | 200µL | 0.13 µg/µl | 26 µg | 52 | (0.052) |
| Hy 150'Da | 50µL Σ 250µL | 0.1 µg/µl | 5 µg | 10 | (0.010) |
| 250 µL is diluted to 500µL by pH shift pH 7.4/ 2.4 and precipitated by NaCl 2M. | | | | | |

### Dialysis procedure

Dialyse-tube, Spectra/Pore Membrane MWCO 6-9,000 Record No 132645 was used.

The tube was softened in distilled water for 1h. The tube was then cut and a dialyse-clamp was fitted at one edge of the tube. 500 µl was filled in the bag. The filling was done with a sterile micro-pipette. The other edge of the tube was fitted with a dialyse-clamp, and the resulting bag was dialysed for 30 h. Then the integrity of the membrane was controlled. The dialysed solution was aspirated with a sterile micro-pipette and the volume determined (µl).

### Preparation steps

200µL of the plasmid solution 1 (0.13 µg/µl) 26 µg was thawed at 24 °C. Positively charged Hy was prepared. The pH of Solution 2 was checked and found to be pH 8.33. The pH-value of the Hy solutions were studied to obtain a positive 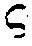 -potential of the Hy structure, see Table 3.

**Table 3.**

| **Acidification of Hy** | | | | | | | |
|---|---|---|---|---|---|---|---|
| Solution 2 in µL | 1 M HCl in µL | Hy in µg/ml | HCl in M | pH_{Prim} | pH₅ₘᵢₙ | pH_{30 min} | pH₃₀ₜᵢₘ |
| 2000 | 500 | 83.2 | 0.2 | 0.76 | ND | 0.68 | 0.81 |
| 2000 | 1000 | 69.3 | 0.33 | 5.19* | 2.28 | 2.93 | 2.9 |
| 2000 | 1500 | 59.4 | 0.42 | 0.37 | ND | 0.36 | 0.47 |
| 2000 | 2000 | 52 | 0.5 | 0.32 | ND | 0.26 | 0.39 |

| | | | | | | | |
|---|---|---|---|---|---|---|---|
| *the pH-value deviates from expected values and was therefore controlled. | | | | | | | |

**Table 4.**

| **Control of deviating pH** | | | | |
|---|---|---|---|---|
| Solution 2 in µL | 1 M HCl in µL | Hy in µg/ml | HCl in M | pH_{Prim} |
| 2000 | 1000 | 69.3 | 0.33 | 0.85 |
| 1000 | 50 | 99.0 | 0.047 | 1.18 |
| 1000 | 25 | 101.4 | 0.024 | 1.69 |

In example 1 it was found that in weak acid solutions the ζ-potential changes from a negatively to a positively charged structure (ζ-potential. from - 69.3 to + 8.9-+ 16.9mV). The results were not consistent that is when the acidity goes towards a lower pH < pH 1.75 the measured ξ-potential indicate an increased negative charged structure of Hy (-4.4, -5.1, -17.9) See table 5.

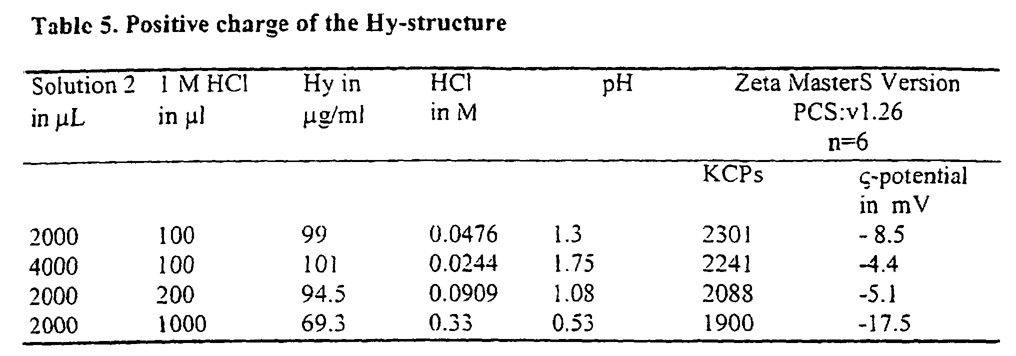

Hy present in strong acid-solutions should be avoided due to a breakdown of the Hy structure and to a loss of the acetylgroup. The time to form a complex between Hy and different plasmid in strong acid solution should therefore be limited to around 30 min.

### Solution to form a complex:

Hy solution, pH 1.75, was used in the studies to form a complex with the plasmid. The samples were dialysed within 30 min against 0.15 M NaCl to rise the pH.

In this study 101 µg/ml Hy, pH 1.75, was chosen for preparing the compressed complex, although the measured 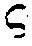 -potential was negative. In solution 94.5 µg/ml Hy, pH 1.08 some measured values were positive but the values of 101 µg/ml Hy, pH 1.75, were more even contributed.

101 µg/ml Hy, pH 1.75, (0.11 x 101.4 = 11. 1 µg Hy) or 110 µl Hy 11.1 µg Hy was mixed with Solution 1, Plasmid solution (pRc/CMV-CAT , 0.13 µg/µl) or 200µL pRc/CMV-CAT , 26.0 µg plasmid added within 30 min

Solution 3, 2 M NaCl 50pl add distilled water 150µl

Final solution Σ 510µl

The final solution 510 µl was incubated for 1 hour at 25°C and then dialysed agains Solution 3.1, 0.15 M NaCl. The dialysis procedure and the membrane was checked twice daily, at 9 p.m. and 4 a.m.

Day 1 (6 h); Day 2 (24 h); Day 3 (24 h); Day 4 (24 h); Day 5 (13 h), Σ 91 h.

The Hy-plasmid complex was formulated to contain (510 µl):

| | |
|---|---|
| pRc/CMV-CAT | 26 µg / 50.98 µg/ml / 12.0 nM |
| Hy (150 000 Dalton) | 11.1 µg / 21.76 µg/ml / 14.5 nM |

### Physical-chemical evaluation

Solution 1, plasmid pRc/CMV in 0.15 M NaCl (1+1) 130/2 µg/ml 30.57/2ρM).

Solution 2. Hya 150 000 Dalton 104 µg/ml (69.3ρM) in distilled water.

The final solution of the complex - (pRc/CMV-CAT+ Hy) 50.98+21.76 µg/ml in 0.15 m NaCl.

These were examined by Dynamic Light Scattering, Malvem Instruments England Zeta Master Version PCS: v1.26. The examined volume was ≅ 500 µL.

**Table 6.**

| **Diameter by Volume (Diameter in nm.) of the Hy-plasmid-complex compared with plasmid pRc/CMV and Hy 150 000 Dalton.** | | | |
|---|---|---|---|
| Solution used | Concentration in µg/ml | nM | Diameter by volume in nm |
| By (150 000 Dalton) in water | 104 | 69.3 | 16.4¹⁾ |
| pRc/CMV-CAT in 0.15 M NaCl (1+1) | 65 | 15.25 | 87.1 |
| | | | Xₙ₌₆ |
| Complex-(pRc/CMV-CAT-Hy) in 0.15 M NaCl | 50.98+21.76 | 12.0+145 | 7.5 |
| | | | Xₙ₌₆ |

| | | | |
|---|---|---|---|
| ¹⁾*16.4* too low intensity of the laser for measurement | | | |

The plasmid pRc/CMV-CAT has been compressed from 87.1 nm to 7.5 nm.

The methods involve acidification, forming a complex, salting and dialyse treatment, at a molar ratio of plasmid/Hy 150 000 Dalton 0.83-0.85:1.

Dynamic light scattering identifies the diameter of the complex pRc/CMV-CAT- Hy (7.5nm).

Positive charging of the Hy- structure (Hy _{pH 8.8} ζ-potential -51 to -69mV → Hy _{pH 1.75} 1.6±2.3mV),

pH-shift (pH < 2→ pH 6) and an ionic concentration change of NaCl 2 M to 0.075 M forms the complex.

It is shown that the compressed state of the complex is stable, as the determinations were performed after 3 months storage at 5-8°C.

### Example 8: Preparation of plasmid-Hy complex according to the invention

A plasmid-Hy complex was prepared for use in transfection studies in cells, and for physicochemical characterisation after three (3) months of storage.

The preparation was done with a newly prepared plasmid (conc. 0.67 µg/ml) with the following changes; a new molar ratio plasmid/Hy 0.1128 (molar ratio plasmid / Hy 0.085), batch size 1020 µl (520µl), pH 1.65 and 1.8 (pH 1.75 and 1.08).

The complex formation is based on a positive charging of the Hyaluronan (Hy) structure (Hy_{Dest. W.} 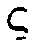 -61.5±8.5mV/ Hy 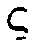 _{PH 1.65} -2.4 ± 2.2mV, Hy_{Dest. W.} 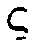 -61.5 ± 8.5mV/ Hy 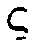 _{pH 1.80} - 4.0mV± 1.9 mV) The complex is formed by a pH-shift (pH ≤2 / ≅6,) and an ionic concentration change (NaCl 2 M /0.075M). The Plasmid-Hy complex is determined after 3 months of storage at 5-8°C and characterised by a plasmid compression of the plasmid from 356 (98.9 -507) nm at pH-shift 1.65 ≥ 6.0 to 84.41 nm at pH-shift 1.80 ≥ 6.0 to 69.0 nm identified by dynamic light scattering (see Table 7).

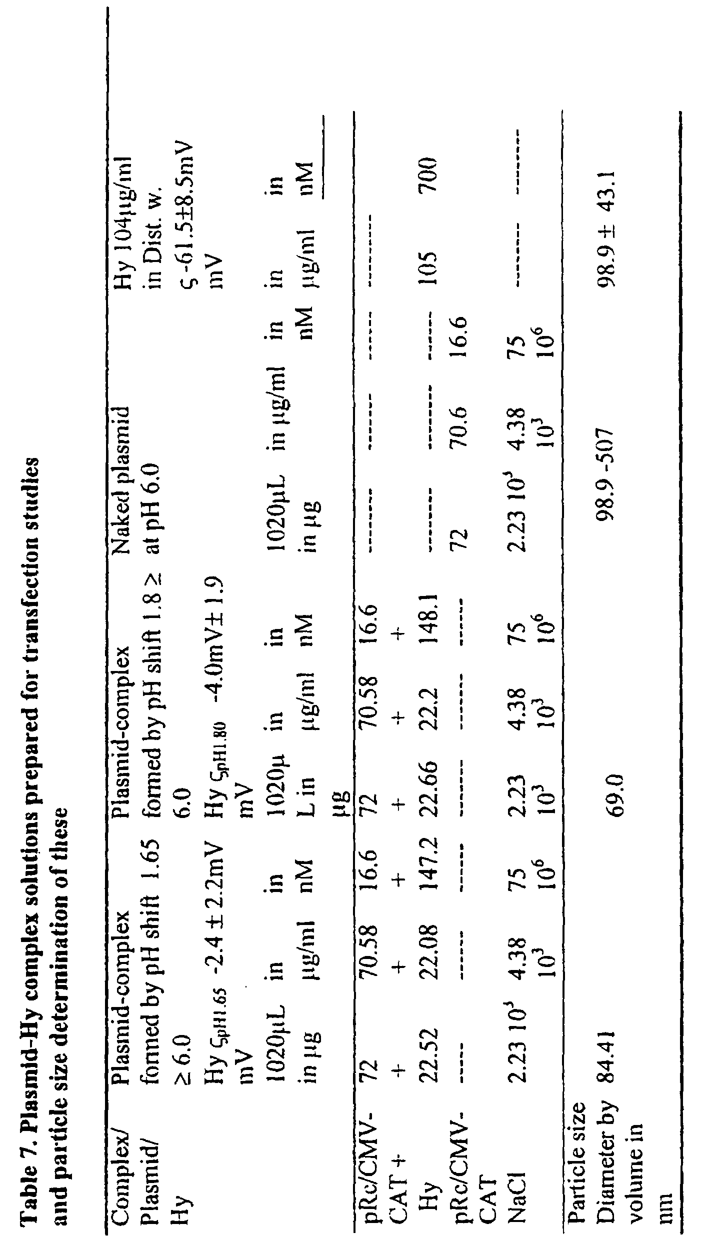

An optimal molar ratio for the plasmid compression was not found. After 3 months storage at 5 - 8°C, the molar ratio plasmid/Hy 0. 1128 resulted in compression from 98.9-507 nm to 69.0 at pH 1.8 and to 84.41 nm at pH 1.65. For a molar ratio plasmid/Hy 0.085, the plasmid was compressed from 87 nm to 7.5 nm at pH 1.75. There were however indications that the new plasmid was impure, which explains that a size distribution with a peak at 98.9 and one peak at 507 nm was found.

### Example 9. Manufacture of rhGH-Hy complex according to the invention

This example illustrates the compression of rhGH. By changing pH to a strong acid-solution (pH 1.5) Hy becomes charged stretching out from a curled cylinder to a straight line. At this pH, rhGH is easily soluble. The size of the rhGH particles is moderated by the speed of the pH-change and the ionic concentration change from 2 M to 0.15 M NaCl. The pH-interval used is 7 - 1.5 - 5.0 (pI) at a constant ratio of rhGH: Hy. Hy stabilises the dispersion in changing its structure back to curled structure at about pH 6 to 7.

The preparations were assayed with HI-HPLC. The particle size of the compressed and the None compressed rhGH was determined by light scattering, Z-master, after storage at 5 - 8°C for 30 days. The diameter of the measured particles is given in means of six measurements.

20.78 mg Hy with a molar mass of 150 KDa was dissolved in 900 µl distilled water and was allowed to react for more than 1 hour. The pH 9.2 of the Hy-solution was adjusted to pH 1.51 with 50 µl water and 50 µl 1 M HCl.

30.65 mg of lyophilised rhGH was dissolved in the Hy-solution. The molar ratio rhGH: Hy is 10 : 1. In portions of 10 µl 1 M HCl was added to obtain a clear solution and to change pH in the solution from 1.7 to 1.48 at that pH the solution was clear. The final volume was 1000 µl. The solutions were divided in two parts and transferred in two bags prepared as a dialyse tube of MWCO 6 - 8000, Spectra Pore R. softened in a tris buffer 10 mM pH 7.8 for 24 hours.

### 9.1 Non-compressed rhGH

30.7 mg rhGH was dissolved in 990 µl distilled water pH 7.4 the final volume of the solution pH 7.0 was adjusted to 1000 µL. The solution was divided in two parts and transferred in two bags (Bag1, Bag2) softened in a Tris buffer 10 mM pH 7.8 for 24 hours.

### 9.2 Dialysis procedure

The tubes were dialysed in Tris buffer 10 mM pH 7.9. The solution, volume 600 ml, was exchanged 3 times. See Table 8.

**Table 8.**

| **Dialyse Procedure MWCO 6-8000, Spectra Pore R** | | | | | | | | | |
|---|---|---|---|---|---|---|---|---|---|
| | | Compressed rhGH | | | | None-compressed rhGH | | | |
| Experiment # Dialyse time in hour | pH in Dialyse Solution | 4₁ (Bag 1) | | 4₁ (Bag 2) | | 4, (Bag 1) | | 4, (Bag 2) | |
| | | pH | Ocular/volume in µl | pH | Ocular/volume in µl | pH | Ocular/volume in µl | pH | Ocular/volume in µl |
| 0 | 7.9 | 1.5 | Clear/500 | 1.5 | Clear/500 | 1.5 | Clear 500 | 1.5 | Clear 500 |
| 1 | 7.4 | N.D. | precipitate | N.D | precipitate | N.D. | Clear | N.D. | Clear |
| 19 | 7.4 | N.D. | Cake in Solution | N.D. | Particles in Solution | N.D. | Clear | N.D. | Clear |
| 42 | 7.8 | 7.4 | Cake in Solution 300 | 7.4 | Particles in solution 750 | 7.6 | Clear/200 | 7.6 | Clear/200 |

The above-prepared compressed rhGH and non-compressed rhGH were used for analytical assay (*in situ* determined biological activity and for particle sizing. The concentrations used for particle sizing of compressed rhGH was 7.6 mg/ml and 9.3 mg/ml and for non-compressed rhGH 15 mg/ml. The measurements, means of six measurements were done at 25°C.

HI-HPLC of the compressed and the none-compressed rhGH. HI-HPLC assay is commonly used to determine biological activity of rhGH

**Table 9.**

| **Analysis of rhGH** | | | |
|---|---|---|---|
| Preparation | Compressed rhGH | | None-compressed rhGH |
| Experiment # | 4₁ Bag 1 | 4₁ Bag 2 | 4 (Bag 1+2) |
| Quantitative Assay, mg/ml | 7.6 | 9.3 | 15.2 |
| Monomer in % | 97.3 | 93.8 | 98.3 |
| LMWG,% | 0.8 | 0.7 | 0.7 |
| Clipped Forms in % | 1.9 | 5.5 | 0.9 |
| Retention Time, min | Approved | Approved | Approved |
| pH in dialysis-tube | 7.4 | 7.4 | 7.6 |
| Appearance in dialysise-tube | cake | particles | clear |

The values obtained for compressed rhGH, 4₁ Bag 1 and 4₁ Bag 2, Table 9, are within limits for an approved biological activity. Values are also of the same magnitude as for the untreated peptide, non-compressed rhGH. This suggests that the procedure to compress rhGH does not change the biological activity of rhGH as determined with HI-HPLC-method.

Particle sizing of Compressed and of None-Compressed rhGH was performed by light scattering, Z-master.

**Table 10.**

| **Particle sizing** | | | |
|---|---|---|---|
| Preparation | Particle size of compressed rhGH diluted 1:10 Diameter by volume in nm | | Particle size of None-compressed rhGH diluted 1:10 Diameter by volume in nm |
| Experiment # | 4₁ Bag 1 | 4₁ Bag 2 | 4 Bag 1+2 |
| Colloidal solution | 23.1 | 23.3 | 75 |
| Cake in equilibrium with a solute | N/A | 114 | N/A |

The values obtained of the diameter by volume for non-compressed rhGH and compressed rhGH indicate that for compressed rhGH the diameter is changed from 75 nm to 23 nm and that a cake or particles are obtained. This suggests that the procedure to compress rhGH significantly reduces the particle size of rhGH.

### Example 10. Biological activity of rhGH-Hy complex and non-treated rhGH

14.9 mg Hy with a molar mass of 150 KDa was dissolved in 1200 µl 1 M Na₂SO₄ and was allowed to react for more than 1 hour

The pH of the Hy-solution pH 7.6 was adjusted to pH 1.51 with 100 µl water and 330 µl HCl 1 M to a clear solution. HCl was added slowly, and when passing pH 3.8 a precipitate was observed.

22 mg of lyophilised rhGH was dissolved in the Hy-solution. The molar ratio rhGH: Hy is 10: 1. In portions of 10µl 1M HCl was added to obtain a clear solution. The starting pH was 2.77. 40µl of 1 M HCl was added to change pH in the solution from 2.77 to 1.52.

The solution was not totally clear. A loss of the solution was obtained when transferred to the dialyse bag. The final volume was 1240 µl. The solutions were divided in two parts and transferred in two bags prepared as a dialyse tube of MWCO 6-8000, Spectra Pore R. softened in a Tris buffer 10 mM pH 7.8.

### 10.1 Non-compressed rhGH

22 mg rhGH was dissolved in 990 µl distilled water pH 7.4. The final volume of the solution was adjusted to 1240 µL. The solution was divided in two parts and transferred in two bags (Bag 1, Bag2) softened in a tris buffer 10 mM pH 7.8.

### 10.2 Dialyse procedure

The tubes were dialysed in Tris buffer 10-mM pH 7.9. The solution, volume 600-ml, was exchanged 3 times. See Table 11.

**Table 11.**

| **Dialysis Procedure MWCO 6-8000, Spectra Pore R** | | | | | | | | | |
|---|---|---|---|---|---|---|---|---|---|
| EXPERlMENT# | | Compressed rhGH | | | | Non-compressed rhGH | | | |
| | | 5₁(Bag 1) | | 5₁(Bag 2) | | 5 (Bag 1) | | 5 (Bag 2) | |
| Dialyse time in hour | pH in Dialyse Solution | pH | Ocular/volume in µl | pH | Ocular/volume in µl | pH | Ocular/volume in µl | pH | Ocular/volume in µl |
| 0 | 7.9 | 1.5 | Clear/620 | 1.5 | Clear/620 | 1.5 | Clear 620 | 1.5 | Clear 620 |
| 19 | 7.4 | N.D. | precipitate | N.D | precipitate | N.D. | Clear | N.D. | Clear |
| 24 | 7.5 | N.D. | Cloudy | N.D. | precipitate | N.D. | Clear | N.D. | Clear |
| 42 | 7.6 | 7.3 | almost clear 420 | 7.3 | almost clear 1100 | 7.6 | Clear/550 | 7.4 | Clear/450 |

The above-prepared compressed rhGH and non-compressed rhGH are used for analytical assay (in situ determined biological activity), in vivo biological activity and for particle sizing. The concentrations used for particle sizing compressed rhGH were 5,3 mg and 1.5 mg and for non-compressed rhGH 5-10 mg. The sizing is given in means of ten measurements and was done at 18, 25 and 30°C.

### 10.3 Analysis of rhGH

HI-HPLC analysis of compressed and of non-compressed rhGH was performed. HI-HPLC assay is commonly used to determine the biological activity of rhGH.

**Table 12.**

| **Analysis of rhGH** | | | | | |
|---|---|---|---|---|---|
| Preparation | Compressed rhGH | | | Non-compressed rhGH | |
| Experiment # | 4₁ (Bag 1) | 4₁ (Bag 2) | 5₁ (Bag 1+2) | 4 (Bag1+2) | 5 (Bag 1+2) |
| Quantitative Assay mg/ml | 7.6 | 9.3 | 5.3 | 15.2 | 14.2 |
| Monomer,% | 97.3 | 93.8 | 100 | 98.3 | 98 |
| LMWG,% | 0.3 | 0.7 | < 0.3 | 0.7 | 0.6 |
| Clipped forms,% | 1.9 | 5.5 | < 0.2 | 0.9 | 0.2 |
| Retention Time, min | Approved | Approved | Approved | Approved | Approved |
| pH in dialyse-tube | 7.4 | 7.4 | 7.3 | 7.6 | 7.4 |
| Appearance in dialyse-tube | cake | particles | almost clear | clear | clear |
| Volume of clear solution in µl | 300 | 750 | 1520 | 400 | 1000 |

The values obtained for compressed rhGH, 4₁(Bag 1), 4₁ (Bag 2), and 5₁ (Bag1+2), Table 12, are within limits for an approved biological activity. Values are also of the same magnitude as for the untreated peptide, non-compressed rhGH (4 and 5 Bag1+2) this suggests that the procedure to compress rhGH does not change the biological activity of rhGH as determined using the HI-HPLC-method.

The hydrodynamic radius and the molecular weight of native rhGH and the rhGH-Hy complex was determined with DynaPro 801. See Table 13.

**Table 13.**

| **Properties of compacted rhGH** | | | | |
|---|---|---|---|---|
| Compact rhGH structure | | | | |
| Assayed concentration in mg/ml | 1.5 | | 5.3 | |
| Temp. at size measurements°C | 18 | 25 | 25 | 30 |
| Molar mass of the polymers in kDa | 165 | 142 | 716 | 1125 |
| Hydrodynamic radii of the polymers in nm | 5.3 | 4.9 | 9.4 | 11.4 |
| Number of compacted units rhGH in the polymers | 7 | 6 | 32 | 50 |
| Estimated hydrodynamic radii of one compacted rhGH-unit in the polymer in nm | 0.70 | 0.77 | 0.22 | 0,29 |

The radius of compressed rhGH is smaller than non-compressed rhGH. That is, at a concentration of 5.3 mg/ml one compressed unit of rhGH is estimated to have a hydrodynamic radii of 0.22- 0.29 nm and at 1.5 mg/ml 0.70-0.77 nm. At a concentration of 14.5 mg/ml non-compressed rhGH is estimated to have a hydrodynamic radii of 2.4 nm. The number of units of compressed rhGH contained in a polymer is found to depend on the concentration of the solution. Upon dilution no agglomerate of the polymers were found.

### 10.4 Biological activity

The biolgical activity of compressed rhGH and non-compressed rhGH was evaluated after parental injections in hypophysis-ectomised (Hx) rats.

The dose response of 0.04 IU/ml and 0.16 IU/ml of compressed rhGH and of non-compressed rhGH were evaluated in groups of 10 Hx rats. A group was treated with a placebo solution (Bovine albumin 12.5 ml (200 mg/ml) was diluted with isotonic NaCl to 1000 ml. This solution, containing 1.25% albumin, was used as placebo and to dilute the solutions of compressed rhGH and non-compressed rhGH to doses for animal trials.)

Solutions from the dialyse tubes containing compressed rhGH, 5.3 mg/ml, and non-compressed rhGH 14.2 mg/ml, were aseptically diluted with distilled water and assayed; Compressed rhGH, 0.23 mg/ml or 0.69 IU/ml and non-compressed rhGH to 0.48 mg/ml or 1.45 IU/ml.

Injection solutions of compressed and of non-compressed rhGH were prepared by dilution with Diluent 1.25 % Albumin solution to desired strength, low dose 0.04 IU/ml and high dose 0.16 IU/ml. Diluent 1.25 % Albumin solution was used as placebo.

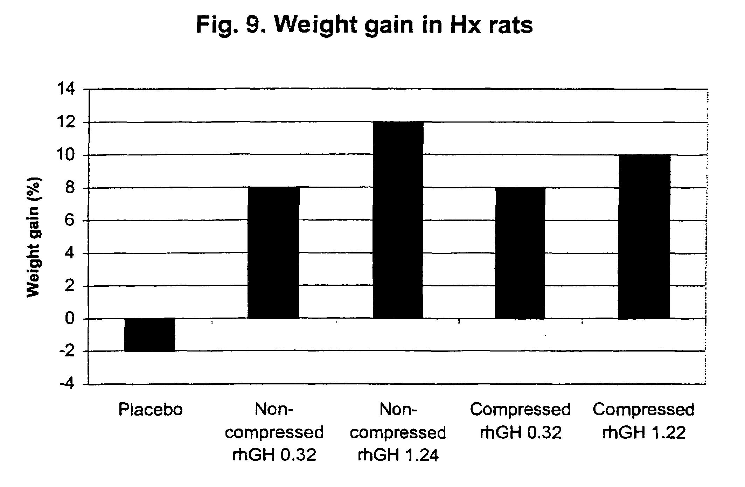

Figure 9 illustrates the weight gained in procent of administered dose (treatment of rhGH in i.u./kg body weight for different doses). Dose response of compressed and non-compressed rhGH in % weight gain is demonstrated Table 14 after subcutaneous injection in Hx rats.

**Table 14**

| **Weight Gain in % given as mean of ten H-x rats. (X** _{**n = 10**} **in %)** | | | | | |
|---|---|---|---|---|---|
| *Preparation* | *Compressed rhGH* | | *Non-compressed rhGH* | | *Placebo* |
| Dose in IU/ kg Body-weight | 0.32 | 1.22 | 0.32 | 1.24 | 0 |
| % Weight Gain | 8 | 10 | 8 | 12 | -2 |

A dose response in % weight gain is demonstrated for compressed, and for non-compressed rhGH in Figure 9 and Table 14. For compressed rhGH, an 8 % weight gain was registered for the 0.32 IU/kg dose, and a 10 % weight gain for the 1.22 IU/kg dose. For non-compressed rhGH, an 8 % weight gain for 0.32 IU/kg, and 12 % for 1.24 lU/kg was registered. For Placebo, no weight gain was obtained.

The dose response obtained for compressed rhGH in Hx rats suggests that full biological effect be obtained after the compression of the rhGH structure.

## Claims

1. A complex between a biomolecule and hyaluronic acid, **characterized in that** the biomolecule is reversibly compressed to less than about 10% of its original size, and that the complex is formed by means of hydrophobic interactions between the components in an acid electrolyte solution.

2. A complex according to claim 1, **characterized in that** said hyaluronic acid is hyaluronic acid having a molecular weight of less than about 400 kDa, preferably in the interval of 60 to 360 kDa.

3. A complex according to claim 1, **characterized in that** said hyaluronic acid is hyaluronic acid having a molecular weight of about 150 kDa.

4. A complex according to any of claims 1-3, **characterized in that** said biomolecule is a peptide.

5. A complex according to any of claims 1-3, **characterized in that** said biomolecule is a protein.

6. A complex according to any of claims 1-3, **characterized in that** said biomolecule is a glycoprotein

7. A complex according to any of claims 1-3, **characterized in that** said biomolecule is a polynucleotide.

8. A complex according to claim 4, **characterized in that** said peptide is chosen among the following groups of substances: hormones, neuropeptides, signal peptides, peptide antibiotics, peptide antigens, antibodies and naturally occurring or synthetic amino acid polymers.

9. A complex according to claim 4, **characterized in that** said peptide is chosen among the following substances: insulin, growth hormone, human growth hormone, glucagon, corticotropin, oxytocin, bradykinin, thyrotropin-release factor and thyrotropin, enkephalins, and peptide antibiotics.

10. A complex according to claim 7, **characterized in that** said polynucleotide is chosen among the following: plasmides, DNA, RNA, cDNA, mRNA, recombinant DNA, recombinant RNA, and combinations thereof.

11. A method for manufacturing a complex of a biomolecule and hyaluronic acid, **characterized in that** the method comprises the following steps:
a) dissolving hyaluronic acid in a solution containing an acid and an electrolyte solution,
b) dissolving the biomolecule in the dissolved hyaluronic acid and an acid solution, and
c) dialysing the solution formed in b) at about neutral pH.

12. A method according to claim 11, **characterized in that** the acid in step a) and b) is chosen among hydrochloric acid, sulphuric acid, phosphoric acid and acetic acid and the pH is in the range of 1.0 to 3.0.

13. A method according to claim 11 or 12, **characterized In that** the electrolyte solution of step a) contains cations chosen among NH₄⁺, K⁺, Na⁺, Ca²⁺, Mg²⁺, Zn²⁺, Fe²⁺, Fe³⁺ and anions such as sulphate, nitrate, chloride, hydroxide and acetate.

14. A method according to any of claims 11-13, **characterized in that** the pH in step c) is in the interval of 5.5 to 7.5.

15. A method according to any of claims 11-14, **characterized in that** said hyaluronic acid is hyaluronic acid having a molecular weight of less than about 400 kDa, preferably In the interval of 60 to 360 kDa.

16. A method according to claim 15, **characterized in that** said hyaluronic acid is hyaluronic acid having a molecular weight of about 150 kDa.

17. The use of insulin for the manufacture of a complex of reversibly compressed insulin and hyaluronic acid, wherein the insulin is reversibly compressed to less than about 10% of its original size, for use as a medicament for the treatment of diabetes.

18. The use of insulin for the manufacture of a complex of reversibly compressed insulin and hyaluronic acid, wherein the insulin is reversibly compressed to less than about 10% of its original size, for use as an orally administered medicament for the treatment of diabetes.

19. The use of insulin for the manufacture of a complex of reversibly compressed insulin and hyaluronic acid, wherein the insulin is reversibly compressed to less than about 10% of its original size, for use as a parentally administered medicament for the treatment of diabetes.

20. The use of growth hormone for the manufacture of a complex of reversibly compressed growth hormone and hyaluronic acid, wherein the growth hormone is reversibly compressed to less than about 10% of its original size, for use as a medicament for the treatment of growth hormone deficiencies.

21. The use of growth hormone for the manufacture of a complex of reversibly compressed growth hormone and hyaluronic acid, wherein the growth hormone is reversibly compressed to less than about 10% of its original size, for use as a medicament for the prevention of transplant rejections.

22. The use of heparin or bioactive derivatives thereof for the manufacture of a complex of reversibly compressed heparin or derivatives thereof and hyaluronic acid, wherein the heparin or bioactive derivatives thereof is reversibly compressed to less than about 10% of its original size, for use as a medicament.

## Patentansprüche

1. Komplex zwischen einem Biomolekül und Hyaluronsäure, **dadurch gekennzeichnet , dass** das Biomolekül reversibel auf weniger als etwa 10% seiner ursprünglichen Größe komprimiert ist und dass der Komplex durch hydrophobe Wechselwirkungen zwischen den Komponenten in einer Säureelektrolytlösung gebildet wird.

2. Komplex nach Anspruch 1, **dadurch gekennzeichnet, dass** die Hyaluronsäure Hyaluronsäure mit einem Molekulargewicht von weniger als etwa 400 kDa, vorzugsweise im Intervall von 60 bis 360 kDa, ist.

3. Komplex nach Anspruch 1, **dadurch gekennzeichnet, dass** Hyaluronsäure Hyaluronsäure mit einem Molekulargewicht von etwa 150 kDa ist.

4. Komplex nach einem der Ansprüche 1 bis 3, **dadurch gekennzeichnet, dass** das Biomolekül ein Peptid ist.

5. Komplex nach einem der Ansprüche 1 bis 3, **dadurch gekennzeichnet, dass** das Biomolekül ein Protein ist.

6. Komplex nach einem der Ansprüche 1 bis 3, **dadurch gekennzeichnet, dass** das Biomolekül ein Glycoprotein ist.

7. Komplex nach einem der Ansprüche 1 bis 3, **dadurch gekennzeichnet, dass** das Biomolekül ein Polynucleotid ist.

8. Komplex nach Anspruch 4, **dadurch gekennzeichnet, dass** das Peptid unter den folgenden Substanzgruppen ausgewählt ist: Hormone, Neuropeptide, Signalpeptide, Peptidantibiotika, Peptidantigene, Antikörper und natürlich vorkommende oder synthetische Aminosäurepolymere.

9. Komplex nach Anspruch 4, **dadurch gekennzeichnet, dass** das Peptid unter den folgenden Substanzen ausgewählt ist: Insulin, Wachstumshormon, humanes Wachstumshormon, Glucagon, Corticotropin, Oxytocin, Bradykinin, Thyreotropinreleasing-Faktor und Thyreotropin, Enkephaline und Peptidantibiotika.

10. Komplex nach Anspruch 7, **dadurch gekennzeichnet, dass** das Polynucleotid unter den folgenden ausgewählt ist: Plasmide, DNA, RNA, cDNA, mRNA, rekombinante DNA, rekombinante RNA und Kombinationen davon.

11. Verfahren zur Herstellung eines Komplexes aus einem Biomolekül und Hyaluronsäure, **dadurch gekennzeichnet, dass** das Verfahren die folgenden Stufen umfasst:
a) Lösen von Hyaluronsäure in einer Lösung, die eine Säure und eine Elektrolytlösung enthält,
b) Auflösen des Biomoleküls in der Lösung mit gelöster Hyaluronsäure und Säure und
c) Dialysieren der in b) gebildeten Lösung bei etwa neutralem pH.

12. Verfahren nach Anspruch 11, **dadurch gekennzeichnet, dass** die Säure in Stufe a) und b) unter Salzsäure, Schwefelsäure, Phosphorsäure und Essigsäure ausgewählt ist und der pH im Bereich von 1,0 bis 3,0 liegt.

13. Verfahren nach Anspruch 11 oder 12, **dadurch gekennzeichnet , dass** die Elektrolytlösung von Stufe a) Kationen, die unter NH₄⁺, K⁺, Na⁺, Ca²⁺, Mg²⁺, Zn²⁺, Fe²⁺, Fe³⁺ ausgewählt sind, und Anionen wie zum Beispiel Sulfat, Nitrat, Chlorid, Hydroxid und Acetat enthält.

14. Verfahren nach einem der Ansprüche 11 bis 13, **dadurch gekennzeichnet, dass** der pH in Stufe c) im Intervall von 5,5 bis 7,5 liegt.

15. Verfahren nach einem der Ansprüche 11 bis 14, **dadurch gekennzeichnet, dass** die Hyaluronsäure Hyaluronsäure mit einem Molekulargewicht von weniger als etwa 400 kDa, vorzugsweise im Intervall von 60 bis 360 kDa, ist.

16. Verfahren nach Anspruch 15, **dadurch gekennzeichnet, dass** die Hyaluronsäure Hyaluronsäure mit einem Molekulargewicht von etwa 150 kDa ist.

17. Verwendung von Insulin zur Herstellung eines Komplexes aus reversibel komprimiertem Insulin und Hyaluronsäure, wobei das Insulin auf weniger als etwa 10% seiner ursprünglichen Größe reversibel komprimiert ist, zur Verwendung als Medikament zur Behandlung von Diabetes.

18. Verwendung von Insulin zur Herstellung eines Komplexes aus reversibel komprimiertem Insulin und Hyaluronsäure, wobei das Insulin reversibel auf weniger als etwa 10% seiner ursprünglichen Größe komprimiert ist, zur Verwendung als oral verabreichtes Medikament zur Behandlung von Diabetes.

19. Verwendung von Insulin zur Herstellung eines Komplexes aus reversibel komprimiertem Insulin und Hyaluronsäure, wobei das Insulin auf weniger als etwa 10% seiner ursprünglichen Größe reversibel komprimiert ist, zur Verwendung als parenteral verabreichtes Medikament zur Behandlung von Diabetes.

20. Verwendung von Wachstumshormon zur Herstellung eines Komplexes aus reversibel komprimiertem Wachstumshormon und Hyaluronsäure, wobei das Wachstumshormon auf weniger als etwa 10% seiner ursprünglichen Größe reversibel komprimiert ist, zur Verwendung als Medikament für die Behandlung von Wachstumshormondefizienzien.

21. Verwendung von Wachstumshormon für die Herstellung eines Komplexes aus reversibel komprimiertem Wachstumshormon und Hyaluronsäure, wobei das Wachstumshormon reversibel auf weniger als etwa 10% seiner ursprünglichen Größe komprimiert ist, zur Verwendung als Medikament zur Prävention von Transplantatabstoßungen.

22. Verwendung von Heparin oder bioaktiven Derivaten davon zur Herstellung eines Komplexes aus reversibel komprimiertem Heparin oder Derivaten davon und Hyaluronsäure, wobei das Heparin oder bioaktive Derivate davon reversibel auf weniger als etwa 10% seiner ursprünglichen Größe/ihrer ursprünglichen Größe komprimiert ist/sind, zur Verwendung als Medikament.

## Revendications

1. Complexe entre une biomolécule et un acide hyaluronique, **caractérisé en ce que** la biomolécule est comprimée de manière réversible jusqu'à moins d'environ 10% de sa taille initiale et que le complexe est formé au moyen d'interactions hydrophobes entre les composants présents dans une solution d'électrolytes acide.

2. Complexe selon la revendication 1, **caractérisé en ce que** ledit acide hyaluronique est un acide hyaluronique d'un poids moléculaire de moins d'environ 400 kDa, de préférence compris dans un intervalle de 60 à 360 kDa.

3. Complexe selon la revendication 1, **caractérisé en ce que** ledit acide hyaluronique est un acide hyaluronique d'un poids moléculaire d'environ 150 kDa.

4. Complexe selon l'une quelconque des revendications 1 à 3, **caractérisé en ce que** ladite biomolécule est un peptide.

5. Complexe selon l'une quelconque des revendications 1 à 3, **caractérisé en ce que** ladite biomolécule est une protéine.

6. Complexe selon l'une quelconque des revendications 1 à 3, **caractérisé en ce que** ladite biomolécule est une glycoprotéine.

7. Complexe selon l'une quelconque des revendications 1 à 3, **caractérisé en ce que** ladite biomolécule est un polynucléotide.

8. Complexe selon la revendication 4, **caractérisé en ce que** ledit peptide est choisi parmi le groupe de substances suivant : hormones, neuropeptides, peptides signal, antibiotiques peptidiques, antigènes peptidiques, anticorps et polymères d'acides aminés naturels ou synthétiques.

9. Complexe selon la revendication 4, **caractérisé en ce que** ledit peptide est choisi parmi le groupe de substances suivant : insuline, hormone de croissance, hormone de croissance humaine, glucagon, corticotropine, oxytocine, bradykinine, facteur de libération de la thyrotropine et thyrotropine, enképhalines et antibiotiques peptidiques.

10. Complexe selon la revendication 7, **caractérisé en ce que** ledit polynucléotide est choisi parmi les polynucléotides suivants : plasmides, ADN, ARN, ADNc, ARNm, ADN recombinant, ARN recombinant et des combinaisons de ceux-ci.

11. Procédé de fabrication d'un complexe comprenant une biomolécule et un acide hyaluronique, **caractérisé en ce que** le procédé comprend les étapes suivantes :
a) la dissolution de l'acide hyaluronique dans une solution contenant un acide et une solution d'électrolytes,
b) la dissolution de la biomolécule dans l'acide hyaluronique dissous et une solution acide, et
c) la dialyse de la solution formée en b) à un pH approximativement neutre.

12. Procédé selon la revendication 11, **caractérisé en ce que** l'acide de l'étape a) et b) est choisi parmi l'acide chlorhydrique, l'acide sulfurique, l'acide phosphorique et l'acide acétique et le pH est dans une gamme de 1,0 à 3,0.

13. Procédé selon la revendication 11 ou 12, **caractérisé en ce que** la solution d'électrolytes de l'étape a) contient des cations choisis parmi le NH₄⁺, le K⁺, le Na⁺, le Ca²⁺, le Mg²⁺, le Zn²⁺, le Fe²⁺, le Fe³⁺ et des anions tels que le sulfate, le nitrate, le chlorure, l'hydroxyde et l'acétate.

14. Procédé selon l'une quelconque des revendications 11 à 13, **caractérisé en ce que** le pH de l'étape c) est compris dans un intervalle de 5,5 à 7,5.

15. Procédé selon l'une quelconque des revendications 11 à 14, **caractérisé en ce que** ledit acide hyaluronique est un acide hyaluronique d'un poids moléculaire de moins d'environ 400 kDa, de préférence compris dans un intervalle de 60 à 360 kDa.

16. Procédé selon la revendication 15, **caractérisé en ce que** ledit acide hyaluronique est un acide hyaluronique d'un poids moléculaire d'environ 150 kDa,

17. Utilisation de l'insuline pour la fabrication d'un complexe constitué d'insuline comprimée de manière réversible et d'acide hyaluronique, où l'insuline est comprimée de manière réversible jusqu'à moins d'environ 10% de sa taille initiale, destiné à être utilisé en tant que médicament pour le traitement du diabète.

18. Utilisation de l'insuline pour la fabrication d'un complexe constitué d'insuline comprimée de manière réversible et d'acide hyaluronique, où l'insuline est comprimée de manière réversible jusqu'à moins d'environ 10% de sa taille initiale, destiné à être utilisé en tant que médicament administré par voie orale pour le traitement du diabète.

19. Utilisation de l'insuline pour la fabrication d'un complexe constitué d'insuline comprimée de manière réversible et d'acide hyaluronique, où l'insuline est comprimée de manière réversible jusqu'à moins d'environ 10% de sa taille initiale, destiné à être utilisé en tant que médicament administré par voie parentérale pour le traitement du diabète.

20. Utilisation de l'hormone de croissance pour la fabrication d'un complexe constitué d'hormone de croissance comprimée de manière réversible et d'acide hyaluronique, où l'hormone de croissance est comprimée de manière réversible jusqu'à moins d'environ 10% de sa taille initiale, destiné à être utilisé en tant que médicament destiné au traitement des déficits en hormone de croissance.

21. Utilisation de l'hormone de croissance pour la fabrication d'un complexe constitué d'hormone de croissance comprimée de manière réversible et d'acide hyaluronique, où l'hormone de croissance est comprimée de manière réversible jusqu'à moins d'environ 10% de sa taille initiale, destiné à être utilisé en tant que médicament destiné à la prévention du rejet de greffe.

22. Utilisation de l'héparine ou de dérivés bioactifs de celle-ci pour la fabrication d'un complexe constitué d'héparine ou de dérivés bioactifs de celle-ci comprimés de manière réversible et d'acide hyaluronique, où l'héparine ou les dérivés bioactifs de celle-ci sont comprimés de manière réversible jusqu'à moins d'environ 10% de leur taille initiale, destiné à être utilisé en tant que médicament.
